(19) Europäisches Patentamt
European Patent Office
Office européen des brevets

(11) Numéro de publication : **0 190 058**
**B1**

(12) # FASCICULE DE BREVET EUROPÉEN

(45) Date de publication du fascicule du brevet : **23.12.87**

(51) Int. Cl.[4] : **C 07 K 5/10**, A 61 K 37/02

(21) Numéro de dépôt : **86400010.4**

(22) Date de dépôt : **06.01.86**

(54) **Nouveaux dérivés peptidiques à structure polycyclique azotée, leur procédé de préparation et les compositions pharmaceutiques qui les contiennent.**

(30) Priorité : **07.01.85 FR 8500140**

(43) Date de publication de la demande :
**06.08.86 Bulletin 86/32**

(45) Mention de la délivrance du brevet :
**23.12.87 Bulletin 87/52**

(84) Etats contractants désignés :
**AT BE CH DE FR GB IT LI LU NL SE**

(56) Documents cités :
**US-A- 4 353 823**
**CHEMICAL ABSTRACTS, vol. 83, 1975, page 611, abrégé no. 114937e, Columbus, Ohio, US; & JP - A - 75 13 373 (DAIGO NUTRITIVE CHEMICALS LTD.) 12.02.1975**

(73) Titulaire : **ADIR**
**22, rue Garnier**
**F-92200 Neuilly-sur-Seine (FR)**

(72) Inventeur : **Vincent, Michel**
**8 allée du Prunier Hardy**
**F-92220 Bagneux (FR)**
Inventeur : **Rémond, Georges**
**9 avenue des Etats-Unis**
**F-78000 Versailles (FR)**
Inventeur : **Cudennec, Claude**
**23 bis avenue de Circourt**
**F-78170 La Celle-St-Cloud (FR)**

**EP 0 190 058 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne de nouveaux tétrapeptides, leur préparation et les compositions pharmaceutiques qui les contiennent.

On connaît de nombreux tétrapeptides naturels ou synthétiques et notamment la tuftsine (Thr—Lys—Pro—Arg) modificateurs de la réponse biologique. Certains analogues de la tuftsine ont été décrits en particulier dans les brevets US N° 4 353 823 et DE 2 343 034, et ces analogues possèdent une activité stimulante de la phagocytose comparable en général à celle de la tuftsine.

La demanderesse a maintenant découvert de nouveaux tétrapeptides dont les propriétés immunomodulatrices sont supérieures à celles de la tuftsine.

Plus spécifiquement, l'invention concerne des dérivés tétrapeptides de formule générale :

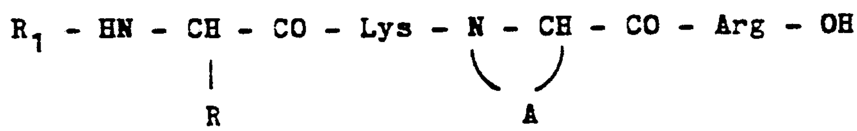

(I)

dans laquelle :

R représente :

un atome d'hydrogène,

un radical alkyle comportant de 1 à 5 atomes de carbone en chaîne droite ou ramifiée, éventuellement substitué par un groupement hydroxy, amino, mercapto, méthylthio, méthylsulfoxyde, carboxy ou guanidino, ou

un radical benzyle ou (indolyl-3) méthyle éventuellement substitué par un groupement hydroxyle,

$R_1$ représente un atome d'hydrogène ou un groupement alkyle contenant de 1 à 4 atomes de carbone.

Lys et Arg représentent respectivement les restes lysyle et arginyle engagés dans des liaisons peptidiques,

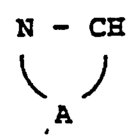

représente

une structure bicyclique de formule :

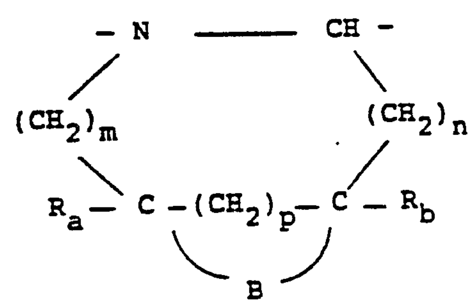

où

m est égal à 1 ou zéro,

n et p représentent zéro, 1 ou 2,

$R_a$ et $R_b$ représentent un atome d'hydrogène ou peuvent former ensemble une liaison directe quand p = 0,

B représente une chaîne alkylène $(CH_2)_q$ où q est égal à 2, 3 ou 4 ou une structure insaturée $(—CH=CH—)_2$ quand p = 0 et $R_a$ et $R_b$ forment ensemble une liaison,

avec la réserve que la somme de m, n, p et q est un nombre entier compris entre 3 et 6, ou

la tétrahydro-1,2,3,4-bétacarboline,

leurs énantiomères, épimères et diastéréoisomères,

ainsi que leurs sels d'addition à un acide ou une base pharmaceutiquement acceptable.

Actuellement, parmi les composés de formule I, on préfère ceux dans lesquels la structure cyclique

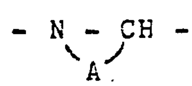

représente l'indoline, l'isoindoline, la tétrahydroquinoléine, la tétrahydroisoquinoléine, le perhydroindole, le perhydroisoindole, la perhydroisoquinoléine, la perhydroquinoléine, le perhydrocyclopenta [b] pyrrole, l'aza-2-bicyclo [2.2.2] octane, l'aza-2 bicyclo [2.2.1] heptane, la tétrahydro-1,2,3,4 bétacarboline.

Parmi les acides que l'on peut ajouter aux composés de formule I pour former un sel d'addition, on peut citer à titre d'exemple, les acides chlorhydrique, sulfurique, phosphorique, tartrique, malique, maléique, fumarique, oxalique, méthanesulfonique, éthanesulfonique, camphorique, citrique, etc...

Comme bases pouvant salifier les composés de formule I on pourra utiliser des hydroxydes de sodium, potassium, calcium ou d'aluminium, des carbonates de métaux alcalins ou alcalinoterreux ou des bases organiques comme la triéthylamine, la benzylamine, la diéthanolamine, la tert.butylamine, la dicyclohexylamine, l'arginine, etc...

L'invention s'étend aussi au procédé d'obtention des composés de formule I, caractérisé en ce que l'on protège la fonction aminée d'un dérivé de formule II :

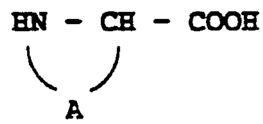

(II)

dans laquelle A avec les atomes de carbone et d'azote auxquels il est rattaché, a les mêmes significations que dans la formule I, par un radical tertiobutoxycarbonyle (tBoc) sous l'action du carbonate de ditertiobutyle en un dérivé de formule III :

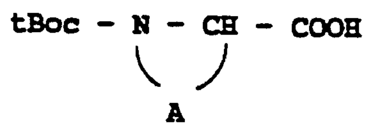

(III)

dans lequel A avec les atomes de carbone et d'azote auxquels il est attaché, a les mêmes significations que dans la formule I, que l'on condense avec le Nω-nitroarginate de méthyle ($Arg(NO_2)OCH_3$) pour obtenir un dérivé de formule IV :

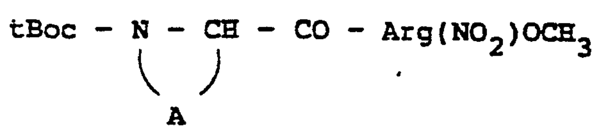

(IV)

dans lequel A, avec les atomes de carbone et d'azote auxquels il est attaché, a les mêmes significations que dans la formule I, que l'on déprotège ensuite par l'acide trifluoroacétique en un dérivé de formule V :

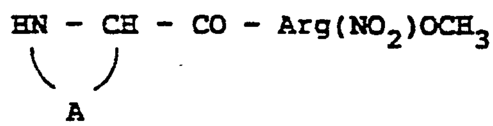

(V)

dans lequel A, avec les atomes de carbone et d'azote auxquels il est attaché, a les mêmes significations que dans la formule I, qui est ensuite condensé avec la N-tertiobutoxycarbonyl Nω-benzyloxycarbonyl lysine ou (tBoc)Lys(Z) pour obtenir un composé de formule VI :

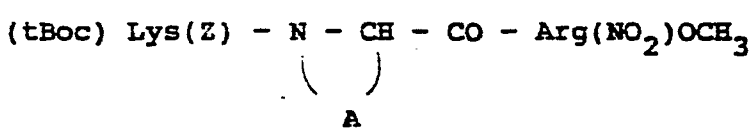

(VI)

dans lequel A, avec les atomes de carbone et d'azote auxquels il est attaché, a les mêmes significations que dans la formule I qui est soumis à l'action de l'acide trifluoroacétique et converti en un dérivé de formule VII :

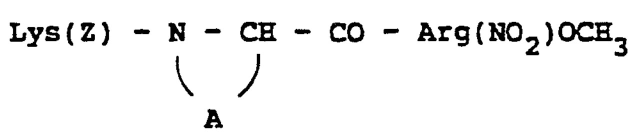

(VII)

dans lequel A, avec les atomes de carbone et d'azote auxquels il est attaché, a les mêmes significations que dans la formule I, que l'on condense avec un dérivé de formule VIII :

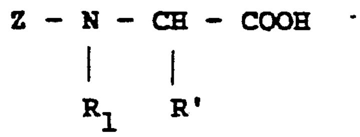

(VIII)

dans lequel

Z signifie un groupement protecteur des fonctions aminées et en particulier le groupe benzyloxycarbonyle ;

$R_1$ a la même signification que dans la formule I ;

R′ représente un atome d'hydrogène, un radical benzyle ou (indolyl-3) méthyle éventuellement substitué par un groupement hydroxyle, ou un radical alkyle, comportant de 1 à 5 atomes de carbone, en chaîne droite ou ramifiée, éventuellement substitué par un groupement hydroxy, mercapto, méthylthio, N-nitro ou NN-bisbenzyloxycarbonyl guanidino ou un groupement carboxylate de benzyle,

pour conduire à un dérivé de formule IX :

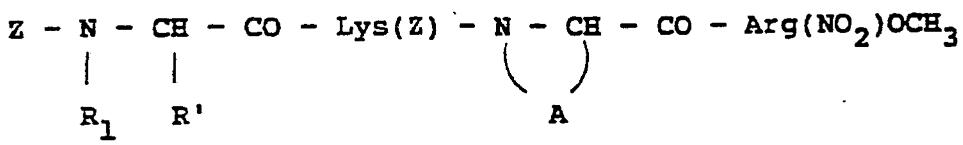

(IX)

dans laquelle A, avec les atomes de carbone et d'azote auxquels il est attaché, et $R_1$ ont les mêmes significations que dans la formule I et R′ les mêmes significations que dans la formule VIII, que l'on saponifie en un dérivé de formule X :

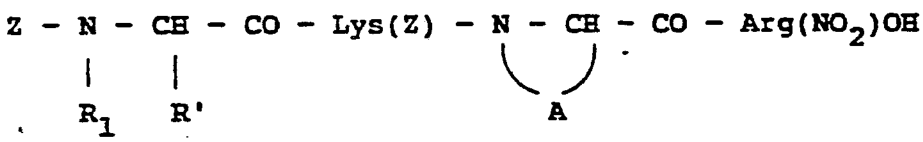

(X)

dans laquelle A, avec les atomes de carbone et d'azote auxquels il est attaché, et $R_1$ ont les mêmes significations que dans la formule I et R′ les mêmes significations que dans la formule VIII, qui est ensuite déprotégé par hydrogénolyse, dans un solvant polaire acide en présence d'un catalyseur d'hydrogénation, en un dérivé de formule I, que l'on peut si l'on désire :

soit salifier par un acide ou une base pharmaceutiquement acceptables.

soit séparer en ses isomères, puis, si nécessaire, salifier par un acide ou une base pharmaceutiquement acceptables.

Les dérivés de formule IX et X sont nouveaux et font partie de l'invention au même titre que les dérivés de formule I, dont ils constituent les intermédiaires de synthèse.

Les composés de formule I sont doués de propriétés pharmacologiques intéressantes.

Ils stimulent notamment l'activité phagocytaire des macrophages et augmentent l'activité des cellules N. K. « Tueuses spontanées ». Administrés à la souris porteuse d'un mélanome, ils inhibent de façon importante la croissance de ce mélanome. Ils assurent une promotion des défenses immunitaires chez des animaux infectés par des souches bactériennes pathogènes.

Ces activités sont liées aux propriétés immunomodulatrices des composés de l'invention qui trouvent leur application, en thérapeutique animale ou humaine, dans le traitement des cancers, des affections d'origine virale, bactérienne ou fongique, des maladies auto-immunes comme le lupus érythémateux ou l'arthrite rhumatoïde et plus généralement dans les maladies résultant d'une diminution ou d'une perturbation des réponses immunitaires naturelles de l'organisme animal ou humain.

L'invention a également pour objet les compositions pharmaceutiques contenant au moins un composé de formule générale I, ou un de ses sels d'addition à un acide ou à une base pharmaceutiquement acceptables, seul ou en combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration parentérale, per ou transcutanée, nasale, oculaire ou respiratoire, et notamment les préparations injectables, les aérosols, les gouttes oculaires ou nasales, les crèmes, pommades, gels dermiques, etc...

La posologie utile varie selon l'âge et le poids du patient, la voie d'administration, la nature de l'indication thérapeutique et des traitements éventuellement associés et s'échelonne entre 1 microgramme et 1 gramme par prise ou par application.

Les exemples suivants illustrent l'invention, et ne la limitent en aucune façon.

Les produits de départ sont connus de la littérature.

Les points de fusion indiqués sont mesurés selon la technique micro-Kofler. Les spectres de résonance magnétique nucléaire du $^{13}C$ ont été enregistrés en utilisant le $D_2O$ comme solvant et le TMS comme référence interne, ceux de $^1H$ RMN ont généralement été enregistrés en utilisant le CD $Cl_3$ comme solvant. Les spectres de masse sont enregistrés après ionisation chimique (gaz réacteur $NH_3$) et détection par un quadripôle.

Exemple 1

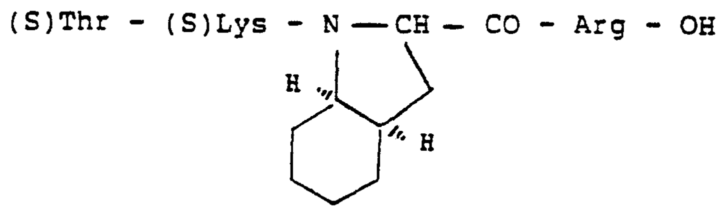

Par commodité, le groupement (2S,3aS,7aS) carbonyl-2 perhydroindole sera dénommé (S)PHI.

Stade A

tBoc(S)PHI—OH ou acide [(tert.butoxycarbonyl-1) (2S,3aS,7aS) perhydroindolyl-2] carboxylique.

Dans un mélange de 60 $cm^3$ de dioxanne et 30 $cm^3$ d'eau, dissoudre 0,03 mole de (S)PHI—OH, obtenu selon Tetrahedron Letters 23, (16) 1677-1680 (1982), refroidir à 0 °C, ajouter 70 $cm^3$ de soude N, puis goutte à goutte une solution de 0,03 mole de carbonate de ditert.butyle dans 100 $cm^3$ de dioxanne. Agiter pendant 30 minutes, à une température comprise entre 0 °C et 5 °C, puis 2 heures à température ambiante.

Evaporer les solvants sous pression réduite. Reprendre le résidu par de l'eau acidifiée par de l'acide citrique jusqu'à pH = 4 et extraire la phase aqueuse par de l'acétate d'éthyle.

Laver la phase organique par une solution aqueuse de chlorure de sodium à 10 %, la sécher sur sulfate de calcium anhydre, filtrer, et la concentrer sous pression réduite. Cristalliser le résidu dans le n.pentane, essorer, sécher.

On obtient le tBoc(S)PHI—OH ;
F° = 134 °C ; Rendement : 82,5 %
Caractéristiques spectrales en I. R. :
$\nu$OH : 2 400-3 200 $cm^{-1}$ ;
$\nu$CO (acide) : 1 750 $cm^{-1}$ ;

$\nu CO(N-\overset{\overset{O}{\|}}{C}-O-)$ : 1 630 $cm^{-1}$

Stade B

tBoc(S)PHI—(S)Arg($NO_2$)$OCH_3$

En utilisant la méthode de W. Konig et R. Geiger, (Ber., 103, 788 (1970)), coupler 0,004 mole de tBoc(S)PHI—OH obtenu au stade précédent avec 0,004 mole de (S)Nω-nitroarginate de méthyle en utilisant le diméthylformamide anhydre comme solvant.

On obtient le tBoc(S)PHI—(S)Arg($NO_2$)$OCH_3$ avec un rendement de 92,5 % sous forme d'une huile utilisée telle quelle dans le stade suivant :
Caractéristiques spectrales en I. R. :
$\nu$NH : 3 100-3 500 $cm^{-1}$ ;
$\nu$CO (ester) : 1 740 $cm^{-1}$ ;
$\nu$CO (amide et $> N-\underset{\underset{O}{\|}}{C}-O-$) : 1 530 et 1 670 $cm^{-1}$ ;

Stade C

(S)PHI—(S)Arg($NO_2$)$OCH_3$

En utilisant la méthode de déprotection par l'acide trifluoroacétique dans le chlorure de méthylène anhydre décrite par B. Gutte et R. B. Merrifield (J. Am. Chem. Soc., 91, 501 (1969)), on obtient quantitativement, à partir de 0,003 52 mole de tBoc(S)PHI—(S)Arg($NO_2$)$OCH_3$ préparé au stade précé-

dent, le (S)PHI—(S)Arg($NO_2$)$OCH_3$ sous forme de trifluoroacétate, dont la pureté est vérifiée par chromatographie sur couche mince. (solvant : $CH_2Cl_2$—MeOH : 9-1 ; Rf = 0,1).

Stade D

tBoc(S)Lys(Z)—(S)PHI—(S)Arg($NO_2$)$OCH_3$

En remplaçant dans le stade B précédent le tBoc(S)PHI—OH par la N-tertbutoxycarbonyl Nω-benzyloxycarbonyl lysine ou tBoc(S)Lys(Z) (0,003 2 mole), et le (S)Arg($NO_2$)$OCH_3$ par le (S)PHI—(S)Arg($NO_2$)$OCH_3$ (0,003 2 mole) préparé au stade précédent, on obtient de la même façon le tBoc(S)lys(Z)—(S)PHI—(S)Arg($NO_2$)$OCH_3$ avec un rendement de 64 %.

Principales caractéristiques $^1$H RMN
3,7 ppm : 3H ($OCH_3$)
5,1 ppm : 2H (benzyle)
7,35 ppm : 5H (aromatiques)
7,8 ppm : 6H (échangeables)

Stade E

(S)Lys(Z)—(S)PHI—(S)Arg($NO_2$)$OCH_3$,

En déprotégeant le tBoc(S)Lys—(S)PHI—(S)Arg($NO_2$)$OCH_3$ par l'acide trifluoroacétique, comme indiqué dans le stade C précédent, on obtient quantitativement le (S)Lys(Z)—(S)PHI—(S)Arg($NO_2$)$OCH_3$ sous forme de trifluoroacétate, dont la pureté est vérifiée par chromatographie sur couche mince (solvant = $CH_2Cl_2$—MeOH : 9-1).

Stade F

(S)(Z)Thr—(S)Lys(Z)—(S)PHI—(S)Arg($NO_2$)$OCH_3$

En condensant, selon la technique indiquée au stade B précédent, le (S)Lys(Z)—(S)PHI—(S)Arg($NO_2$)$OCH_3$ avec la (S)N-benzyloxycarbonyl thréonine (ou (S)(Z)Thr—OH), on obtient de la même façon le (S)(Z)Thr—(S)Lys(Z)—(S)PHI—(S)Arg—$NO_2$$OCH_3$ qui est purifié par chromatographie sur silice (60-230 mesh) en utilisant comme éluant le mélange $CH_2Cl_2$—MeOH (95-5)

Rendement : 67 %.

Analyse centésimale :

Calculé : C 57,20 H 6,74 N 14,29 %
Trouvé : C 57,09 H 6,69 N 14,16 %

Caractéristiques spectrales en
Infra-rouge : νNH,OH : 2 700-3 600 $cm^{-1}$
νCO (amide) : 1 630-1 720 $cm^{-1}$
$^1$H RMN :
2,9 à 5,6 ppm : 10H(CH et $CH_2$)
3,75 ppm : 3H($OCH_3$)
5,1 ppm : 4H (benzyliques)
7,35 ppm : 10H (aromatiques)

Stade G

(S)(Z)Thr—(S)Lys(Z)—(S)PHI—(S)Arg($NO_2$)OH

Dans un mélange de 0,6 $cm^3$ de NaOH 1N, 6 $cm^3$ d'eau et 20 $cm^3$ d'éthanol, introduire 0,000 6 mole de (S)(Z)Thr—(S)Lys(Z)—(S)PHI—(S)Arg($NO_2$)$OCH_3$ et maintenir à température ambiante pendant 5 heures. Concentrer la solution sous pression réduite, reprendre par de l'eau et acidifier par de l'acide chlorhydrique jusqu'à pH = 2. Extraire par de l'acétate d'éthyle. La phase organique prélevée est séchée sur sulfate de Ca anhydre, filtrée, concentrée à sec.

Le produit obtenu est purifié par chromatographie sur silice F254 (éluant $CH_2Cl_2$(MeOH : 80/20) (Rendement : 27 %).

Stade H

(S)Thr—(S)Lys—(S)PHI—(S)Arg—OH

Soumettre 0,000 4 mole de (S)(Z)Thr—(S)Lys(Z)—(S)PHI—(S)Arg($NO_2$)OH obtenu au stade précédent à une hydrogénation catalytique dans 25 $cm^3$ d'acide acétique, sous une pression d'hydrogène de 3 kg/$cm^2$ en présence de 300 mg de charbon palladié à 10 %. Après évaporation du solvant sous pression réduite, le résidu amorphe est repris par 5 $cm^3$ d'eau distillée et séparé par microfiltration.

On obtient le diacétate de (S)Thr—(S)Lys—(S)PHI—(S)Arg—OH (rendement 90 %) que l'on purifie éventuellement par chromatographie haute performance préparative (phase : $SiO_2$—C8 Lichoprep —$RP_8$ Merck N° 9 324 ; éluant $H_2O$-méthanol : 8-2 + 0,02 acide acétique (rendement 60 %)) et lyophilise.

Caractéristiques spectrales en :

Infra-rouge : $\nu$NH et OH : 2 300-3 600 $cm^{-1}$

$\nu$C = O : 1 570-1 760 $cm^{-1}$

RMN ($^1$H) : solvant $CDCl_3$

0,8 à 2,3 ppm : 22H($CH_2$ et CH) + 6H($CH_3$ de $CH_3COOH$)

2,5 à 4,5 ppm : 12H($CH_2$ et CH en $\alpha$ de CO, $\geq$N,—O—)

5,5 ppm : 14H (échangeables).

## Exemple 2

Gly—(S)Lys—(S)PHI—(S)Arg—OH

En remplaçant dans l'exemple 1 stade F, la (S)N-benzyloxycarbonyl thréonine par la N-benzyloxycarbonyl glycine ou (Z)Gly, on obtient de la même façon successivement :

le (Z)Gly—(S)Lys(Z)—(S)PHI—(S)Arg($NO_2$)$OCH_3$ (rendement 70 %) ;

le (Z)Gly—(S)Lys(Z)—(S)PHI—(S)Arg($NO_2$)OH

le Gly—(S)Lys—(S)PHI—(S)Arg—OH (rendement 98 %) qui est lyophilisé sous forme de diacétate.

Caractéristiques spectrales en $^{13}$C RMN :

43,3 ppm : $CH_2$(Gly)

54,1 ppm : CH$\alpha$(Lys)

57,3 ppm : CH$\alpha$(Arg)

63,7 ppm : CH$\alpha$(PHI)

## Exemple 3

(S)NVa—(S)Lys—(S)PHI—(S)Arg—OH

En remplaçant dans l'exemple 1, stade F, la (S)N-benzyloxycarbonyl thréonine, par la (S)N-benzyloxycarbonyl norvaline ou (S)(Z)NVa, on obtient de la même façon successivement les :

(S)(Z)NVa—(S)Lys(Z)—(S)PHI—(S)Arg($NO_2$)$OCH_3$ (rendement 61 %) ;

(S)(Z)NVa—(S)Lys(Z)—(S)PHI—(S)Arg($NO_2$)—OH

(S)NVa—(S)Lys—(S)PHI—(S)Arg—OH (rendement 95 %) ; qui est lyophilisé sous forme de diacétate.

Caractéristiques spectrales en $^{13}$C RMN :

53,8 ppm : CH$\alpha$(Lys)

55,3 ppm : CH$\alpha$(NVa)

57,2 ppm : CH$\alpha$(Arg)

63,4 ppm : CH$\alpha$(PHI)

## Exemples 4 à 7

De la même façon, en remplaçant dans l'exemple 1, stade F, la (S)N-benzyloxycarbonyl thréonine par la (S)N-benzyloxycarbonyl cystéine, le (S)N-benzyloxycarbonyl glutamate de benzyle, la (S)N-benzyloxycarbonyl phénylalanine, et la (R)N-benzyloxy alanine, on obtient respectivement les composés suivants :

Exemple 4 : (S)Cys—(S)Lys—(S)PHI—(S)Arg—OH

lyophilisé sous forme de diacétate (rendement 78 %).

Exemple 5 : (S)Glu—(S)Lys—(S)PHI—(S)Arg—OH

rendement 92 % lyophilisé sous forme de monoacétate.

Principaux fragments caractéristiques en spectrométrie de masse :

m/Z : 583 (invisible), 565, 547, 523, 505, 391, 115.

Exemple 6 : (S)Phe—(S)Lys—(S)PHI—(S)Arg—OH

rendement 94 %, lyophilisé sous forme de diacétate.

Caractéristiques spectrales en $^{13}$C RMN

56,6 ppm : CHα(Phe)
53,2 ppm : CHα(Lys)
63,4 ppm : CHα(PHI)
57,3 ppm : CHα(Arg)

Exemple 7 : (R)Ala—(S)Lys—(S)PHI—(S)Arg—OH

lyophilisé sous forme de diacétate (rendement 91 %).

Caractéristiques spectrales en $^{13}C$ RMN
51,5 ppm : CHα(Ala)
54,0 ppm : CHα(Lys)
57,3 ppm : CHα(Arg)
63,5 ppm : CHα(PHI)

Exemple 8

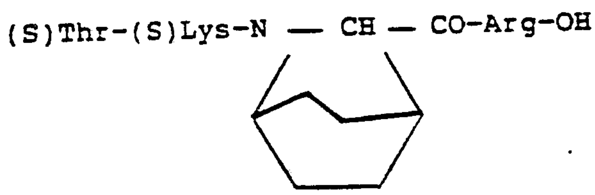

Le radical (S) carbonyl-3 aza-2 bicyclo [2.2.2] octane sera ultérieurement dénommé (S)ABO.

En remplaçant dans l'exemple 1 stade A le (S)PHI—OH par le (S)ABO—OH, préparé selon la méthode décrite dans le brevet européen N° 51020, on obtient de la même façon les composés suivants :

Stade A : tBoc(S)ABO—OH

Rendement 75 % ; F °C :.200 °C ;
Analyse centésimale :

Calculé : C 61,16 H 8,29 N 5,44 %
Trouvé : C 60,85 H 8,35 N 5,53 %

Stade B : tBoc(S)ABO—(S)Arg($NO_2$)$OCH_3$

Stade C : (S)ABO—(S)Arg($NO_2$)$OCH_3$

Stade D : (S)tBocLys(Z)—(S)ABO—(S)Arg($NO_2$)$OCH_3$ Rendement 70 % ;

Stade E : (S)Lys(Z)—(S)ABO—(S)Arg($NO_2$)$OCH_3$

Stade F : (S)(Z)Thr—(S)Lys(Z)—(S)ABO—(S)Arg($NO_2$)$OCH_3$ Rendement 39 % ;

Caractéristiques spectrales :
Infra-rouge : $\nu$(NH,OH) : 3 000-3 600 cm$^{-1}$ ;
$\nu$(CO = O) ; 1 600-1 710 cm$^{-1}$ ;
$\nu(\overset{O}{\overset{\|}{C}}—NH)$ : 1 530 cm$^{-1}$.
RMN ($^1H$)
2,9 à 3,5 ppm : 4H(N—$CH_2$)
3,7 ppm : 3H($OCH_3$)
3,8 à 6 ppm : 5H (α de CO, $\geq$ N)
5,1 ppm : 4H (benzyliques)
2,5 ; 4 ; 6,2 ; 7,5 ppm : protons échangeables (OH et NH).

Stade G : (S)(Z)Thr—(S)Lys(Z)—(S)ABO—(S)Arg($NO_2$)OH

Stade H : (S)Thr—(S)Lys—(S)ABO—(S)Arg—OH

sous forme de diacétate
Rendement 50 % ;

Caractéristiques spectrales en $^{1}H$ RMN (solvant = $D_2O$)
1 à 2,5 ppm : 28H ;
2,7 à 3,5 ppm : 4H( $\geq$ N—$CH_2$) et 1H $\geq$CH—N$<$ ;
3,5 à 5 ppm : 5H(CH en α de O, CO,—N$<$ )

## Exemple 9

Gly—(S)Lys—(S)ABO—(S)Arg—OH

En remplaçant dans l'exemple 8 stade F la (S)(Z)Thr—OH par la (Z)Gly—OH, on obtient successivement de la même façon les :
(Z)Gly—(S)Lys(Z)—(S)ABO—(S)Arg($NO_2$)$OCH_3$ Rendement 51 % ;
(Z)Gly—(S)Lys(Z)—(S)ABO—(S)Arg($NO_2$)OH Rendement 91 % ;
—Gly—(S)—Lys(S)ABO—(S)Arg—OH Rendement 92 % (diacétate)
Caractéristiques spectrales en $^{13}C$ RMN
43,4 ppm : $CH_2$(Gly)
53,0 ppm : CHα(Lys)
57,3 ppm : CHα(Arg)
64,2 ppm : CHα(ABO)

## Exemple 10

(S)NVa—(S)Lys—(S)ABO—(S)Arg—OH

En remplaçant dans l'exemple 8, stade F, la (S)(Z)Thr—OH par la (S)(Z)NVa—OH, on obtient successivement les :
(S)(Z)NVa—(S)Lys(Z)—(S)ABO—(S)Arg($NO_2$)$OCH_3$ Rendement 51 % ;
(S)(Z)NVa—(S)Lys(Z)—(S)ABO—(S)Arg($NO_2$)OH Rendement 88 % ;
(S)NVa—(S)Lys—(S)ABO—(S)Arg—OH Rendement 92 % (diacétate).
Caractéristiques spectrales en $^{13}C$ RMN
52,7 ppm : CHα(Lys)
55,5 ppm : CHα(NVa)
57,2 ppm : CHα(Arg)
64,0 ppm : CHα(ABO)

## Exemples 11 à 14

En opérant comme indiqué dans l'exemple 8, stade F et en remplaçant la (S)N-benzoyloxycarbonyl thréonine par le (S)N-benzyloxycarbonyl tryptophane, la (S) N,N bis-benzyloxycarbonyl arginine, la N,N' bis-benzyloxycarbonyl lysine et la N-benzyloxycarbonyl sarcosine, on obtient de la même façon les composés suivants :

### Exemple 11 : (S)Trp—(S)Lys—(S)ABO—(S)Arg—OH

lyophilisé sous forme de diacétate
rendement 89 %
Caractéristiques spectrales en $^{13}C$ RMN :
52,1 ppm : CHα(Lys)
56,1 ppm : CHα(Trp)
57,2 ppm : CHα(Arg)
63,8 ppm : CHα(ABO)

### Exemple 12 : (S)Arg—(S)Lys—(S)ABO—(S)Arg—OH

lyophilisé sous forme de tétracétate
rendement 94 %
Caractéristiques spectrales en $^{13}C$ RMN
53,2 ppm : CHα(Lys)
55,2 ppm : CHα(Arg)
57,2 ppm : CHα(Arg)
63,7 ppm : CHα(ABO)

### Exemple 13 : (S)Lys—(S)—Lys—(S)ABO—(S)Arg—OH

lyophilisé sous forme de triacétate

rendement 92 %
Caractéristiques spectrales en $^{13}C$ RMN
53,1 ppm : CHα(Lys)
55,4 ppm : CHα(Lys)
57,3 ppm : CHα(Arg)
64,1 ppm : CHα(ABO)

Exemple 14 : Sar—(S)Lys—(S)ABO—(S)Arg—OH

lyophilisé sous forme de diacétate
rendement 97 %
Principaux fragments caractéristiques en spectrométrie de masse :
m/Z : 511, 493, 451, 115, 110

Exemple 15 : Gly—(S)Lys—(S)IND—(S)Arg—OH

En opérant comme dans l'exemple 2, mais en remplaçant au stade A le (S)PHI—OH par la (S)carboxy-2 indoline ou (S)IND—OH, on obtient de la même façon le Gly—(S)Lys—(S)IND—(S)Arg—OH sous forme de diacétate.

Principaux fragments caractéristiques en spectrométrie de masse : m/Z : 487, 445, 302, 274, 260, 242, 186, 168, 118, 115.

Exemples 16 à 19

De la même façon, en utilisant la (S)carboxy-1 isoindoline ou (S)ISI, le carboxy-3 aza-2 bicyclo [2.2.1.] heptane ou ABH—OH, la (S)carboxy-3 tétrahydro-1,2,3,4 bêta-carboline ou (S)THC—OH, la carboxy-1 perhydroisoindole ou PHII—OH, on obtient les composés suivants :

Exemple 16 : Gly—(S)Lys—(S)ISI—Arg—OH

lyophilisé sous forme de diacétate
rendement 91 %
Principaux fragments caractéristiques en spectrométrie de masse : m/Z = 487, 445, 302, 274, 260, 186, 168, 118, 115.

Exemple 17 : Gly—(S)Lys—ABH—(S)Arg—OH

lyophilisé sous forme de diacétate
rendement 94 %
Principaux fragments caractéristiques en spectrométrie de masse : m/Z = 483, 465, 441, 423.

Exemple 18 : Gly—(S)Lys—(S)THC—(S)Arg—OH

obtenu sous forme de diacétate
rendement 90 %
Principaux fragments caractéristiques en spectrométrie de masse : m/Z = 498, 313, 186, 115.

Exemple 19 : Gly—(S)Lys—PHII—(S)Arg—OH

lyophilisé sous forme de diacétate
rendement 95 %
Principaux fragments caractéristiques en spectrométrie de masse : m/Z = 493, 451, 280, 266, 186, 129, 115.

Exemples 20 et 21

En opérant comme indiqué dans l'exemple 3 en remplaçant le (S)PHI—OH par la carboxy-3 perhydroisoquinoléine (ou PHIQ—OH) et le carboxy-2 perhydrocyclopenta [b] pyrrole, (ou PCP—OH) on obtient les composés des exemples suivants :

Exemple 20 : (S)NVa—(S)Lys—PHIQ—(S)Arg—OH

lyophilisé sous forme de diacétate
(rendement 96 %).
Principaux fragments caractéristiques en spectrométrie de masse : m/Z = 550, 549, 508, 507, 393,

375, 228, 138, 115, 100.

Exemple 21 : (S)NVa—(S)Lys—PCP—Arg—OH

lyophilisé sous forme de diacétate (rendement 94 %).

Exemple 22 : (S)Thr—(S)Lys—(S)PCP—(S)Arg—OH

En remplaçant dans l'exemple I stade A le (S)PHI—OH par le (S)PCH—OH, on obtient de la même façon le (S)Thr—(S)Lys—(S)PCP—(S)Arg—OH sous forme de diacétate.

Principaux fragments caractéristiques en spectrométrie de masse : m/Z : 481, 463, 455, 437, 419.

Exemple 23 : (S)Leu—(S)Lys—THQ—(S)Arg—OH

En opérant comme indiqué dans l'exemple 1, mais en remplaçant au stade A, le (S)PHI—OH par la carboxy-2 tétrahydro-1,2,3,4 quinoléine ou THQ—OH, et au stade F la (S)(Z)Thr—OH par la (S)N-benzyloxycarbonyl leucine, on obtient le (S)Leu—(S)Lys—THQ—(S)Arg—OH.

Exemple 24 : (S)Lys—(S)Lys—PHQ—(S)—Arg—OH

En opérant comme précédemment, mais en utilisant au stade A, la carboxy-2 perhydroquinoléine (PHQ—OH) et au stade F la N,$N_\omega$-bis benzyloxycarbonyl lysine, on obtient la (S)Lys—(S)Lys—PHQ—(S)Arg—OH.

Exemple 25 : Gly—(S)Lys—PHQ—(S)Arg—OH

En remplaçant dans l'exemple 2, stade A le (S)PHI—OH par le PHQ—OH, on obtient le Gly—(S)Lys—PHQ—(S)Arg—OH sous forme de diacétate.

Principaux fragments caractéristiques en spectrométrie de masse : m/Z = 525, 507, 483, 465, 138, 115.

Exemple 26 : (S)Met—(S)Lys—(S)THIQ—(S)Arg—OH

En opérant comme précédemment, et en utilisant au stade A, la (S)carboxy-3 tétrahydro-1,2,3,4 isoquinoléine ou (S)THIQ—OH et au stade F la (S)N-benzyloxycarbonyl méthionine, on obtient la (S)Met—(S)Lys—(S)THIQ—(S)Arg—OH, lyophilisé sous forme de diacétate.

Principaux fragments caractéristiques en spectrométrie de masse : m/Z = 533, 288, 260, 274.

Exemple 27 : S-oxyde du (S)Met—(S)Lys—(S)THIQ—(S)Arg—OH

En remplaçant dans l'exemple précédent la (S)N-benzyloxycarbonylméthionine par son S-oxyde, on obtient le S-oxyde du (S)met—(S)Lys—(S)THIQ—(S)Arg—OH sous forme de diacétate.

Principaux fragments caractéristiques en spectrométrie de masse : m/Z = 549, 288, 276, 274.

Etude pharmacologique des composés de l'invention

La capacité des composés de l'invention de stimuler l'activité des cellules immuno-compétentes a été vérifiée in vitro et in vivo.

Exemple 28 : Stimulation de la phagocytose in vitro

In vitro, la technique décrite par Descamps B., 1980 (Ann. Immunol. Inst. Past. 131C, N° 2, p. 10) a été utilisée pour mesurer la stimulation des capacités phagocytaires de macrophages par les composés de l'invention : des macrophages péritonéaux de souris (souche $B_6D_2F_1$) sont ensemencés sur des boîtes de Pétri à raison de $10^4$ cellules par boîte. Après attachement de la culture au milieu, les composés de l'invention sont ajoutés en solution aqueuse à une concentration 25 micromolaire par boîte, puis des globules rouges de mouton opsonisés par des immunoglobulines spécifiques sont introduits. Après 1 heure de contact, les cultutres sont lavées et l'on dénombre les macrophages ayant ingéré plus de 2 globules rouges.

Les composés de l'invention augmentent de 23 % le pouvoir phagocytaire des macrophages par rapport à des cultures témoins. Dans les mêmes conditions, le pouvoir d'activation de la tuftsine est de 15 %.

Exemple 29 : Promotion de l'activité N. K.

Les composés selon l'invention ont également été testés pour leur pouvoir promoteur de l'activité « Tueuse spontanée » (« Natural killer »). Les cellules dotées de ce pouvoir forment la première ligne de défense de l'organisme vis-à-vis de l'envahissement septique, viral ou tumoral.

Pour apprécier leur pouvoir stimulant, des composés selon l'invention ont été étudiés selon la technique Reynolds, et coll. 1981, (J. Immunol. 127, 282).

Les composés sont injectés par voie intraveineuse à la dose de 20 à 50 µg/kg à des souris de souche B6D2F1.

Trois jours après le traitement, les animaux sont sacrifiés, leur rate prélevée et dissociée en ses cellules constituantes qui sont ensemencées en culture en présence de cellules tumorales YAC-1 préalablement marquées au chrome radioactif. A l'issue de l'incubation, le pouvoir destructeur des composés de l'invention est mesuré par la quantité de chrome libérée.

A titre d'exemple, le composé de l'exemple 8, à la dose de 25 µg/kg, induit une augmentation, par rapport au témoin, de la libération de chrome de 15 %, alors que la tuftsine à la dose 40 µg/kg ne provoque qu'une libération de 10 %.

Exemple 30 : Inhibition de croissance du mélanome B 16

Les mélanomes sont des tumeurs cancéreuses sensibles à la réaction du système immunitaire du malade. Ils constituent donc un modèle de choix pour apprécier toute stimulation de défense antitumorale.

Le composé selon l'exemple 1 s'est, par exemple, montré capable de ralentir de 40 % la croissance du mélanome B 16 de la souris, lorsque le produit est administré à raison de 20 g/kg, 3 fois par semaine par voie intrapéritonéale. Dans les mêmes conditions, la tuftsine s'est montrée incapable de favoriser le ralentissement de croissance de la tumeur greffée.

Exemple 31 : Augmentation de la résistance des animaux à l'infection.

Certaines souches bactériennes pathogènes sont capables de tuer l'hôte sain chez qui elles sont inoculées. C'est le cas, par exemple, de Klebsiella pneumoniae, agent responsable de la pneumonie (Parant, M. ; et coll. Proc. Natl. Acad. Sci. USA, 1978, 75, n° 7, 3395).

Le composé de l'exemple 8, par exemple, est capable à la dose de 60 µg par animal de protéger de la mort par infection toutes les souris Swiss, femelles, de 20 à 25 g, auxquelles la souche Klebsiella pneumoniae 7823 est inoculée par voie IP, lorsqu'il est administré 48 heures avant l'infection. Dans les mêmes conditions la tuftsine n'a été capable de sauver que 20 % des animaux.

Exemple 32 : Augmentation de la réponse lymphocytaire aux mitogènes.

Des lectines extraites de plantes sont capables de se substituer in vitro à la stimulation de la prolifération des lymphocytes normalement assurée par des antigènes spécifiques. Ces agents sont des mitogènes lymphocytaires.

Après la mise en contact des lymphocytes avec un tel mitogène, on peut mesurer l'intensité de la prolifération et ainsi mesurer la réactivité immunoprotectrice des animaux traités par un composé (Daguillard, F. Med. Clin. North Am., 56, 293).

Ainsi, des lymphocytes spléniques de souris traitées par 0,5 mg par kg du composé de l'exemple 14 exposés à la Concanavaline A à une concentration de 0,5 mg/ml répondent par une prolifération égale à 1,78 fois celle de lymphocytes d'animaux non traités.

Exemple 33 : Compositions pharmaceutiques

Solution injectable

| | |
|---|---|
| Gly—(S)Lys—(S)PHI—(S)Arg—OH : | 0,050 g |
| eau pour préparation injectable : | 2 $cm^3$ |

Crème dermique

| | |
|---|---|
| (S)Thr—(S)Lys—(S)ABO—(S)Arg—OH | 5 g |
| polypropylèneglycol | 25 g |
| vaseline blanche | 10 g |
| alcool à 95° | 10 g |
| eau purifiée | qsp100 g |

**Revendications** (pour les Etats contractants : BE, CH, DE, FR, GB, IT, LI, LU, NL, SE)

1. Composés de formule générale I :

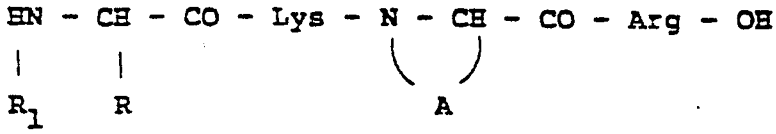

(I)

dans laquelle :

R représente :

un atome d'hydrogène,

un radical alkyle comportant de 1 à 5 atomes de carbone en chaîne droite ou ramifiée, éventuellement substitué par un groupement hydroxy, amino, mercapto, méthylthio, méthylsulfoxyde, carboxy ou guanidino, ou

un radical benzyle ou (indolyl-3) méthyle éventuellement substitué par un groupement hydroxyle,

$R_1$ représente un atome d'hydrogène ou un groupe alkyle de 1 à 4 atomes de carbone.

Lys et Arg représentent respectivement les restes lysyle et arginyle engagés dans des liaisons peptidiques,

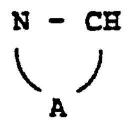

représente

une structure bicyclique de formule :

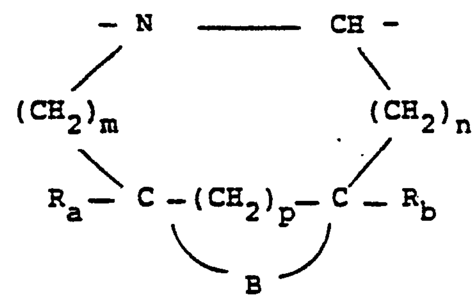

où

m est égal à 1 ou zéro,

n et p représentent zéro, 1 ou 2,

$R_a$ et $R_b$ représentent un atome d'hydrogène ou peuvent former ensemble une liaison directe quand p = 0,

B représente une chaîne alkylène $(CH_2)_q$ où q est égal à 2, 3 ou 4 ou une structure insaturée $(—CH=CH—)_2$ quand p = 0 et $R_a$ et $R_b$ forment ensemble une liaison,

avec la réserve que la somme de m, n, p et q est un nombre entier compris entre 3 et 6, ou

la tétrahydro-1,2,3,4 bétacarboline,

leurs énantiomères, épimères et diastéréoisomères,

ainsi que leurs sels d'addition à un acide ou une base pharmaceutiquement acceptable.

2. Composés selon la revendication 1 dans lesquels la structure cyclique

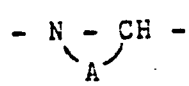

représente l'indoline, l'isoindoline, la tétrahydroquinoléine, la tétrahydroisoquinoléine, le perhydroindole, le perhydroisoindole, la perhydroisoquinoléine, la perhydroquinoléine, le perhydrocyclopenta [b] pyrrole, l'aza-2 bicyclo [2.2.2] octane, l'aza-2 bicyclo [2.2.1] heptane, la tétrahydro-1,2,3,4 bétacarboline, leurs énantiomères, épimères et diastéréoisomères, ainsi que leurs sels d'addition à un acide ou une base pharmaceutiquement acceptable.

3. Composés selon les revendications 1 ou 2 dans lesquelles R représente un atome d'hydrogène, un radical alkyle de 1 à 5 atomes de carbone, en chaîne droite ou ramifiée éventuellement substitué par un groupement hydroxyle, leurs énantiomères, épimères et diastéréoisomères, ainsi que leurs sels d'addition à un acide ou à une base pharmaceutiquement acceptable.

4. Le (S)Thr—(S)Lys—(S)PHI—(S)Arg—OH et ses sels d'addition à un acide ou une base pharmaceutiquement acceptable,

5. Le (S)Thr—(S)Lys—(S)ABO—(S)Arg—OH et ses sels d'addition à un acide ou une base pharmaceutiquement acceptable.

6. Le Gly—(S)Lys—(S)—PHI—(S)Arg—OH et ses sels d'addition à un acide ou une base pharmaceutiquement acceptable.

7. Le Gly—(S)Lys—(S)ABO—(S)Arg—OH et ses sels d'addition à un acide ou une base pharmaceutiquement acceptable.

8. Le (S)NVa—(S)Lys—(S)PHI—(S)Arg—OH et ses sels d'addition à un acide ou une base pharmaceutiquement acceptable.

9. Le (S)NVa—(S)Lys—(S)ABO—(S)Arg—OH et ses sels d'addition à un acide ou une base pharmaceutiquement acceptable.

10. Le Sar—(S)Lys—(S)ABO—(S)Arg—OH et ses sels d'addition à un acide ou une base pharmaceutiquement acceptable.

11. Procédé de préparation des composés de formule I, caractérisé en ce que l'on protège la fonction aminée d'un dérivé de formule II :

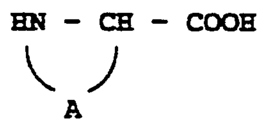

(II)

dans laquelle A, avec les atomes de carbone et d'azote auxquels il est rattaché, a les mêmes significations que dans la formule I, par un radical tertiobutoxycarbonyle (tBoc) sous l'action du carbonate de ditertiobutyle en un dérivé de formule III :

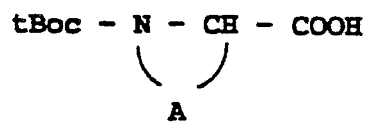

(III)

dans lequel A, avec les atomes de carbone et d'azote auxquels il est attaché, a les mêmes significations que dans la formule I, que l'on condense avec le Nω nitroarginate de méthyle ($Arg(NO_2)OCH_3$) pour obtenir un dérivé de formule IV :

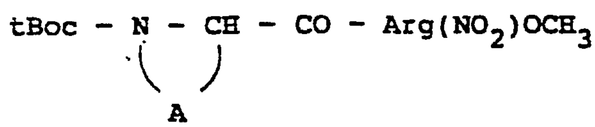

(IV)

dans lequel A, avec les atomes de carbone et d'azote auxquels il est attaché, a les mêmes significations que dans la formule I, que l'on déprotège ensuite par l'acide trifluoroacétique en un dérivé de formule V :

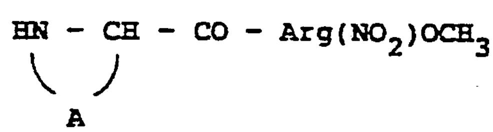

(V)

dans lequel A, avec les atomes de carbone et d'azote auxquels il est attaché, a les mêmes significations que dans la formule I, qui est ensuite condensé avec la N-tert.butoxycarbonyl Nω benzyloxycarbonyl lysine ou (tBoc)Lys(Z) pour obtenir un composé de formule VI :

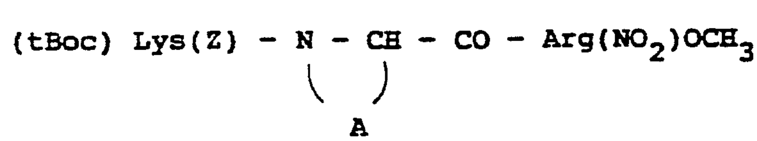

(VI)

dans lequel A, avec les atomes de carbone et d'azote auxquels il est attaché, a les mêmes significations que dans la formule I qui est soumis à l'action de l'acide trifluoroacétique et converti en un dérivé de formule VII :

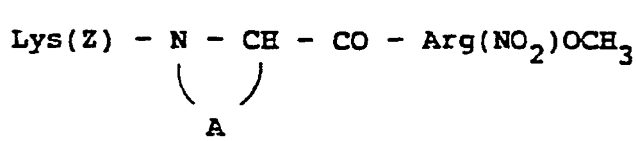

(VII)

dans lequel A, avec les atomes de carbone et d'azote auxquels il est attaché, a les mêmes significations que dans la formule I, que l'on condense avec un dérivé de formule VIII :

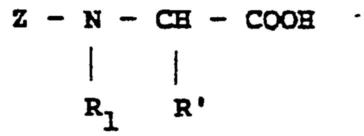

(VIII)

dans lequel

Z signifie un groupement protecteur des fonctions aminées, et en particulier le groupe benzyloxycarbonyle ;

$R_1$ a la même signification que dans la formule I

R' représente un atome d'hydrogène, un radical benzyle ou (indolyle-3) méthyle éventuellement substitué par un groupement hydroxyle, ou un radical alkyle, comportant de 1 à 5 atomes de carbone, en chaîne droite ou ramifiée, éventuellement substitué par un groupement hydroxy, mercapto, méthylthio, N-nitro ou N,N-bisbenzyloxycarbonylguanidino ou un groupement carboxylate de benzyle, pour conduire à un dérivé de formule IX :

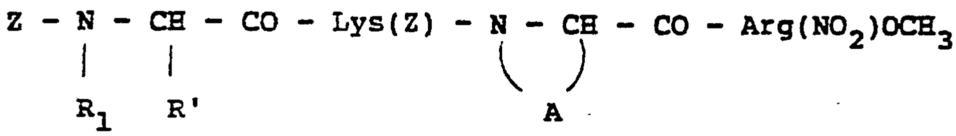

(IX)

dans laquelle A, avec les atomes de carbone et d'azote auxquels il est attaché, et $R_1$ ont les mêmes significations que dans la formule I et R' les mêmes significations que dans la formule VIII, que l'on saponifie en un dérivé de formule X :

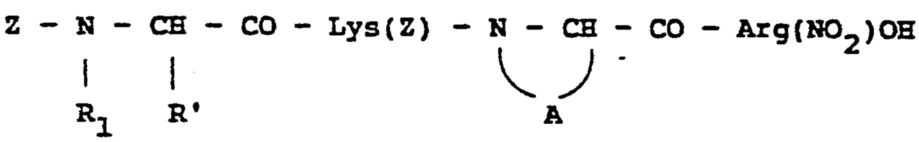

(X)

dans laquelle A avec les atomes de carbone et d'azote auxquels il est attaché, et $R_1$ ont les mêmes significations que dans la formule I et R' les mêmes significations que dans la formule VIII qui est ensuite déprotégé par hydrogénolyse en un dérivé de formule I, que l'on peut si l'on désire :

soit salifier par un acide ou une base pharmaceutiquement acceptables.

soit séparer en ses isomères, puis, si nécessaire, salifier par un acide ou une base pharmaceutiquement acceptables.

12. Composés de structure générale IX selon la revendication 11 utiles pour la préparation des composés selon la revendication 1.

13. Composition pharmaceutique contenant comme principe actif au moins un composé selon les revendications 1 à 10 en combinaison avec un ou plusieurs excipients ou véhicules inertes, non toxiques, pharmaceutiquement acceptables.

**Revendication** (pour l'Etat contractant AT)

Procédé de préparation de composés de formule générale I :

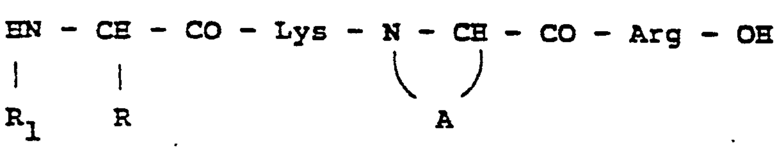

(I)

dans laquelle :

R représente :

un atome d'hydrogène,

un radical alkyle comportant de 1 à 5 atomes de carbone en chaîne droite ou ramifiée, éventuellement substitué par un groupement hydroxy, amino, mercapto, méthylthio, méthylsulfoxyde, carboxy ou guanidino, ou

un radical benzyle ou (indolyl-3) méthyle éventuellement substitué par un groupement hydroxyle.

$R_1$ représente un atome d'hydrogène ou un groupe alkyle de 1 à 4 atomes de carbone.

Lys et Arg représentent respectivement les restes lysyle et arginyle engagés dans des liaisons peptidiques,

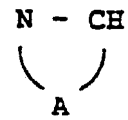

représente

une structure bicyclique de formule :

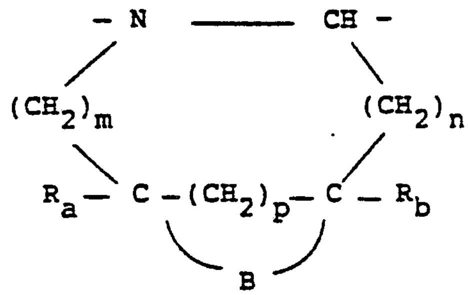

où

m est égal à 1 ou zéro,

n et p représentent zéro, 1 ou 2,

$R_a$ et $R_b$ représentent un atome d'hydrogène ou peuvent se former ensemble une liaison directe quand p = 0,

B représente une chaîne alkylène $(CH_2)_q$ où q est égal à 2, 3 ou 4 ou une structure insaturée $(—CH=CH—)_2$ quand p = 0 et $R_a$ et $R_b$ forment ensemble une liaison,

avec la réserve que la somme de m, n, p et q est un nombre entier compris entre 3 et 6, ou

la tétrahydro-1,2,3,4 bétacarboline,

leurs énantiomères, épimères et diastéréoisomères,

ainsi que leurs sels d'addition à un acide ou une base pharmaceutiquement acceptable, caractérisé en ce que l'on protège la fonction aminée d'un dérivé de formule II :

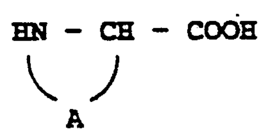

(II)

dans laquelle A, avec les atomes de carbone et d'azote auxquels il est rattaché, a les mêmes significations que dans la formule I, par un radical tertiobutoxycarbonyle (tBoc) sous l'action du carbonate de ditertiobutyle en un dérivé de formule III :

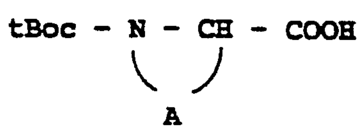

(III)

dans lequel A, avec les atomes de carbone et d'azote auxquels il est attaché, a les mêmes significations que dans la formule I, que l'on condense avec le $N_\omega$ nitroarginate de méthyle $(Arg(NO_2)OCH_3)$ pour obtenir un dérivé de formule IV :

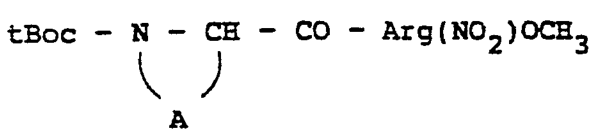

(IV)

dans lequel A, avec les atomes de carbone et d'azote auxquels il est attaché, a les mêmes significations que dans la formule I, que l'on déprotège ensuite par l'acide trifluoroacétique en un dérivé de formule V :

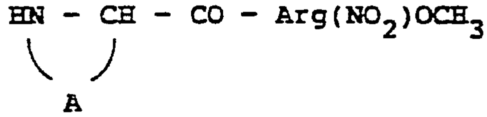

(V)

dans lequel A, avec les atomes de carbone et d'azote auxquels il est attaché, a les mêmes significations que dans la formule I, qui est ensuite condensé avec la N-tert.butoxycarbonyl N benzyloxycarbonyl lysine ou (tBoc)Lys(Z) pour obtenir un composé de formule VI :

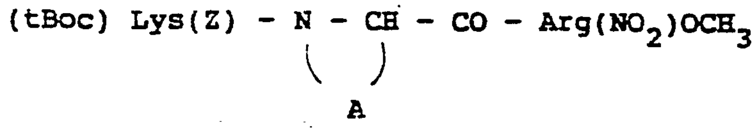
(VI)

dans lequel A, avec les atomes de carbone et d'azote auxquels il est attaché, a les mêmes significations que dans la formule I qui est soumis à l'action de l'acide trifluoroacétique et converti en un dérivé de formule VII :

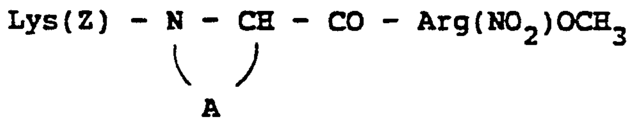
(VII)

dans lequel A, avec les atomes de carbone et d'azote auxquels il est attaché, a les mêmes significations que dans la formule I, que l'on condense avec un dérivé de formule VIII :

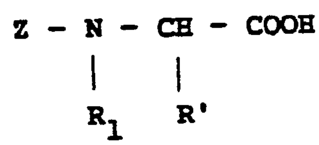
(VIII)

dans lequel

Z signifie un groupemnt protecteur des fonctions aminées, et en particulier le groupe benzyloxycarbonyle ;

$R_1$ a la même signification que dans la formule I

R' représente un atome d'hydrogène, un radical benzyle ou (indolyle-3) méthyle éventuellement substitué par un groupement hydroxyle, ou un radical alkyle, comportant de 1 à 5 atomes de carbone, en chaîne droite ou ramifiée, éventuellement substitué par un groupement hydroxy, mercapto, méthylthio, N-nitro ou N,N-bisbenzyloxycarbonyl guanidino ou un groupement carbòxylate de benzyle,

pour conduire à un dérivé de formule IX :

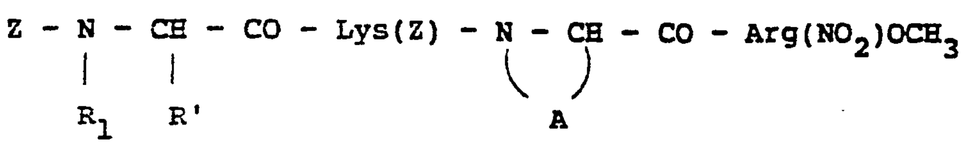
(IX)

dans laquelle A, avec les atomes de carbone et d'azote auxquels il est attaché, et $R_1$ ont les mêmes significations que dans la formule I et R' les mêmes significations que dans la formule VIII, que l'on saponifie en un dérivé de formule X :

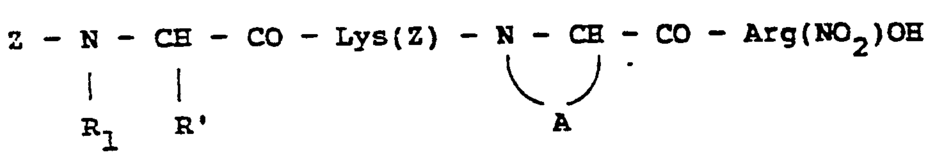
(X)

dans laquelle A avec les atomes de carbone et d'azote auxquels il est attaché, et $R_1$ ont les mêmes significations que dans la formule I et R' les mêmes significations que dans la formule VIII qui est ensuite déprotégé par hydrogénolyse en un dérivé de formule I, que l'on peut si l'on désire :

soit salifier par un acide ou une base pharmaceutiquement acceptables.

soit séparer en ses isomères, puis, si nécessaire, salifier par un acide ou une base pharmaceutiquement acceptables.

**Claims** (for the Contracting States : BE, CH, DE, FR, GB, IT, LI, LU, NL, SE)

1. Compounds of the general formula I

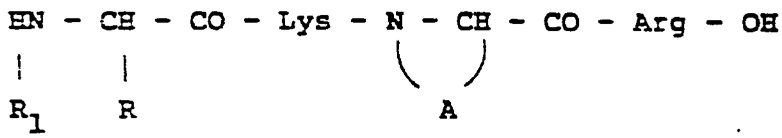 (I)

in which

R represents :

a hydrogen atom,

a straight-chain or branched alkyl radical containing from 1 to 5 carbon atoms, optionally substituted by a hydroxy, amino, mercapto, methylthio, methylsulphoxide, carboxy or guanidino group, or

a benzyl or indol-3-ylmethyl radical optionally substituted by a hydroxy group,

$R_1$ represents a hydrogen atom or an alkyl group containing from 1 to 4 carbon atoms,

Lys and Arg represent, respectively, the lysyl and arginyl residues linked through the peptide bonds,

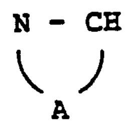

represents

a bicyclic structure of the formula :

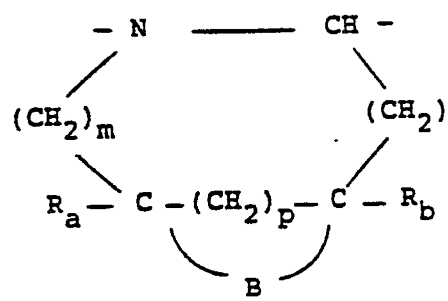

wherein

m represents 1 or 0,

n and p each represents 0, 1 or 2,

$R_a$ and $R_b$ each represents a hydrogen atom or may together form a direct bond when p = 0,

B represents an alkylene chain $(CH_2)q$ in which q represents 2, 3 or 4, or an unsaturated structure $(-CH=CH-)_2$ when p = 0 and $R_a$ and $R_b$ together form a bond,

with the proviso that the sum of m, n, p and q is an integer of from 3 to 6, or,

1,2,3,4-tetrahydro-β-carboline

the enantiomers, epimers and diastereoisomers thereof,

as well as the pharmaceutically acceptable acid or base addition salts thereof.

2. Compounds according to claim 1 in which the cyclic structure

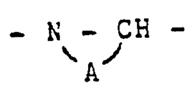

represents indoline, isoindoline, tetrahydroquinoline, tetrahydroisoquinoline, perhydroindole, perhydroisoindole, perhydroisoquinoline, perhydroquinoline, perhydrocyclopenta [b] pyrrole, 2-azabicyclo [2,2,2] octane, 2-azabicyclo [2,2,1] heptane, or 1,2,3,4-tetrahydro-β-carboline, the enantiomers, epimers and diastereoisomers thereof, as well as the pharmaceutically acceptable acid or base addition salts thereof.

3. Compounds according to claims 1 or 2 in which R represents a hydrogen atom, or a straight-chain or branched alkyl radical having from 1 to 5 carbon atoms optionally substituted by a hydroxy group, the enantiomers, epimers and diastereoisomers thereof, as well as the pharmaceutically acceptable acid or base addition salts thereof.

4. (S)Thr—(S)Lys—(S)PHI—(S)Arg—OH and the pharmaceutically acceptable acid or base addition salts thereof.

5. (S)Thr—(S)Lys—(S)ABO—(S)Arg—OH and the pharmaceutically acceptable acid or base addition salts thereof.

6. Gly—(S)Lys—(S)PHI—(S)Arg—OH and the pharmaceutically acceptable acid or base addition salts thereof.

7. Gly—(S)Lys—(S)ABO—(S)Arg—OH and the pharmaceutically acceptable acid or base addition salts thereof.

8. (S)NVa—(S)Lys—(S)PHI—(S)Arg—OH and the pharmaceutically acceptable acid or base addition salts thereof.

9. (S)NVa—(S)Lys—(S)ABO—(S)Arg—OH and the pharmaceutically acceptable acid or base addition salts thereof.

10. Sar—(S)Lys—(S)ABO—(S)Arg—OH and the pharmaceutically acceptable acid or base addition salts thereof.

11. Process for the preparation of compounds of the formula I characterised in that the amine function of a derivative of the formula II

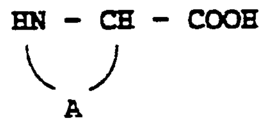

(II)

in which A, together with the carbon and nitrogen atoms to which it is attached, has the same meanings as in formula I, is protected by a tert.-butoxycarbonyl radical (tBoc) by the action of di-tert.-butyl carbonate, resulting in a derivative of the formula III

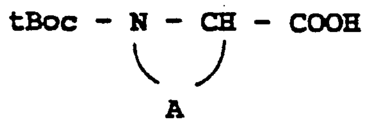

(III)

in which A, together with the carbon and nitrogen atoms to which it is attached, has the same meanings as in formula I, which is condensed with methyl $N_\omega$-nitroarginate ($Arg(NO_2)OCH_3$) to form a derivative of the formula IV

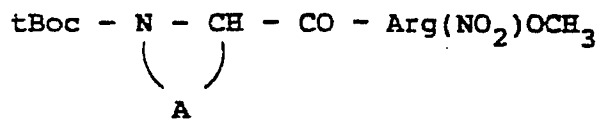

(IV)

in which A, together with the carbon and nitrogen atoms to which it is attached, has the same meanings as in formula I, from which the protecting group is then removed by trifluoroacetic acid to form a derivative of the formula V:

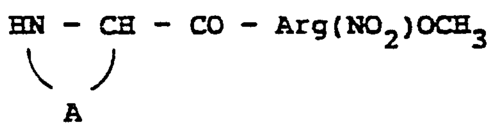

(V)

in which A, together with the carbon and nitrogen atoms to which it is attached, has the same meanings as in formula I, which is then condensed with N-tert.-butoxycarbonyl-$N_\omega$-benzyloxycarbonyllysine or (tBoc)Lys(Z) to form a compound of the formula VI

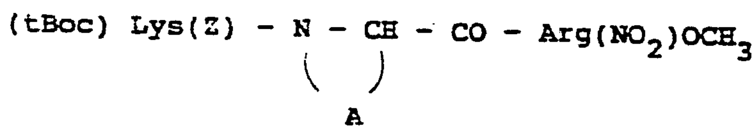

(VI)

in which A, together with the carbon and nitrogen atoms to which it is attached, has the same meanings as in formula I, which is subjected to the action of trifluoroacetic acid and converted into a derivative of the formula VII

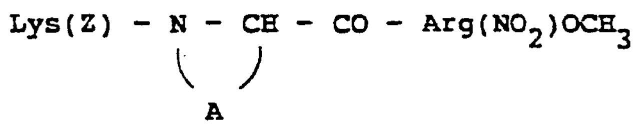

(VII)

in which A, together with the carbon and nitrogen atoms to which it is attached, has the same meanings as in formula I, which is condensed with a derivative of the formula VIII

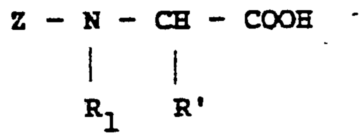

(VIII)

in which

Z represents a protecting group for amine functions, especially a benzyloxycarbonyl group,

$R_1$ has the same meaning as in formula I,

R′ represents a hydrogen atom, a benzyl or indol-3-ylmethyl radical optionally substituted by a hydroxy group, or a straight-chain or branched alkyl radical containing from 1 to 5 carbon atoms that is optionally substituted by a hydroxy, mercapto, methylthio, or N-nitro- or N,N-bisbenzyloxycarbonylguanidino group, or by a benzyl carboxylate group,

to yield a derivative of the formula IX

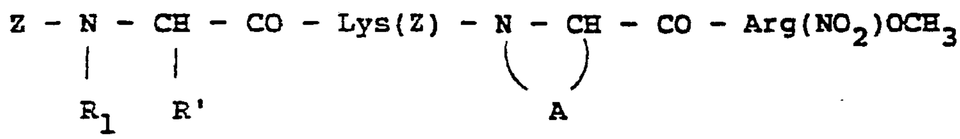

(IX)

in which A, together with the carbon and nitrogen atoms to which it is attached, and $R_1$ have the same meanings as in formula I, and R′ has the same meanings as in formula VIII, which is hydrolysed to form a derivative of the formula X

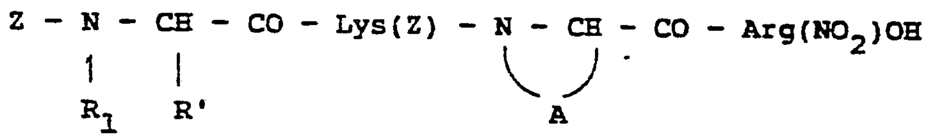

(X)

in which A, together with the carbon and nitrogen atoms to which it is attached, and $R_1$ have the same meanings as in formula I, and R′ has the same meanings as in formula VIII, from which the protecting groups are then removed by hydrogenolysis to form a derivative of the formula I which can then, if desired :

be converted into a salt by a pharmaceutically acceptable acid or base,

be separated into its isomers, then, if necessary, converted into a salt by a pharmaceutically acceptable acid or base.

12. Compounds of the general formula IX according to claim 11 for use in the preparation of compounds according to claim 1.

13. Pharmaceutical composition containing as active substance at least one compound according to claims 1 to 10 in combination with one or more pharmaceutically acceptable inert non-toxic excipients or vehicles.

**Claim** (for the Contracting State AT)

Process for the preparation of compounds of the general formula I

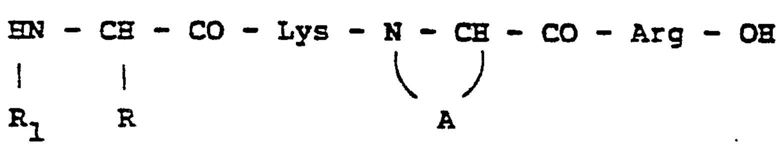

(I)

in which

R represents :

a hydrogen atom,

a straight-chain or branched alkyl radical containing from 1 to 5 carbon atoms, optionally substituted by a hydroxy, amino, mercapto, methylthio, methyl sulphoxide, carboxy or guanidino group, or

a benzyl or indol-3-ylmethyl radical optionally substituted by a hydroxy group,

$R_1$ represents a hydrogen atom or an alkyl group containing from 1 to 4 carbon atoms,

Lys and Arg represent, respectively, the lysyl and arginyl residues linked through the peptide bonds,

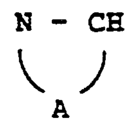

represents

a bicyclic structure of the formula :

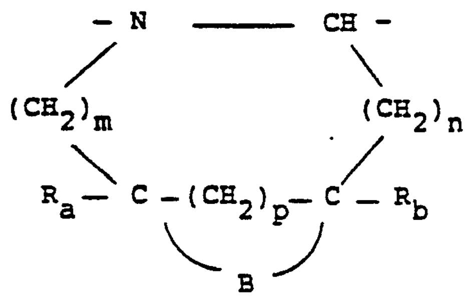

wherein

m represents 1 or 0,

n and p each represents 0, 1 or 2,

$R_a$ and $R_b$ each represents a hydrogen atom or may together form a direct bond when p = 0,

B represents an alkylene chain $(CH_2)q$ in which q represents 2, 3, or 4, or an unsaturated structure $(-CH=CH-)_2$ when p = 0 and $R_a$ and $R_b$ together form a bond,

with the proviso that the sum of m, n, p and q is an integer of from 3 to 6, or,

1,2,3,4-tetrahydro-β-carboline,

the enantiomers, epimers and diastereoisomers thereof;

as well as the pharmaceutically acceptable acid or base addition salts thereof, characterised in that the amine function of a derivative of the formula II

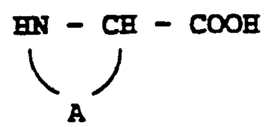

(II)

in which A, together with the carbon and nitrogen atoms to which it is attached, has the same meanings as in formula I, is protected by a tert.-butoxycarbonyl radical (tBoc) by the action of di-tert.-butyl carbonate, resulting in a derivative of the formula III

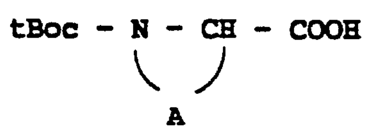

(III)

in which A, together with the carbon and nitrogen atoms to which it is attached, has the same meanings as in formula I, which is condensed with methyl $N_\omega$-nitroarginate ($Arg(NO_2)OCH_3$) to form a derivative of the formula IV

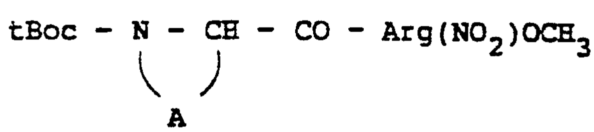

(IV)

in which A, together with the carbon and nitrogen atoms to which it is attached, has the same meanings as in formula I, from which the protecting group is then removed by trifluoroacetic acid to form a derivative of the formula V :

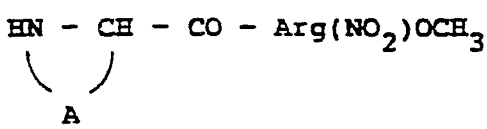

(V)

in which A, together with the carbon and nitrogen atoms to which it is attached, has the same meanings as in formula I, which is then condensed with N-tert.-butoxycarbonyl-N-benzyloxycarbonyllysine or

(tBoc)Lys(Z) to form a compound of the formula VI

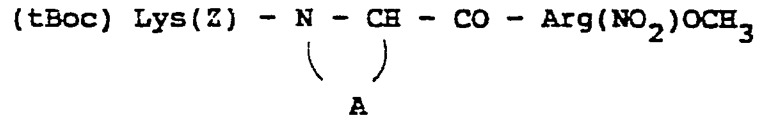

(VI)

in which A, together with the carbon and nitrogen atoms to which it is attached, has the same meanings as in formula I, which is subjected to the action of trifluoroacetic acid and converted into a derivative of the formula VII

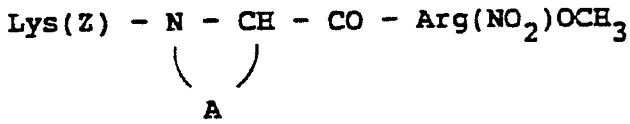

(VII)

in which A, together with the carbon and nitrogen atoms to which it is attached, has the same meanings as in formula I, which is condensed with a derivative of the formula VIII

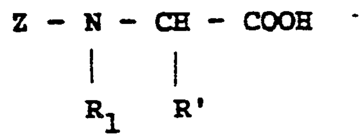

(VIII)

in which

Z represents a protecting group for amine functions, especially a benzyloxycarbonyl group,

$R_1$ has the same meaning as in formula I,

R' represents a hydrogen atom, a benzyl or indol-3-ylmethyl radical optionally substituted by a hydroxy group, or a straight-chain or branched alkyl radical containing from 1 to 5 carbon atoms that is optionally substituted by a hydroxy, mercapto, methylthio, or N-nitro- or N,N-bisbenzyloxycarbonylguanidino group, or by a benzyl carboxylate group,

to yield a derivative of the formula IX

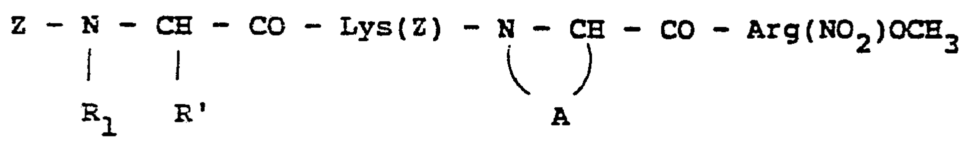

(IX)

in which A, together with the carbon and nitrogen atoms to which it is attached, and $R_1$ have the same meanings as in formula I, and R' has the same meanings as in formula VIII, which is hydrolysed to form a derivative of the formula X

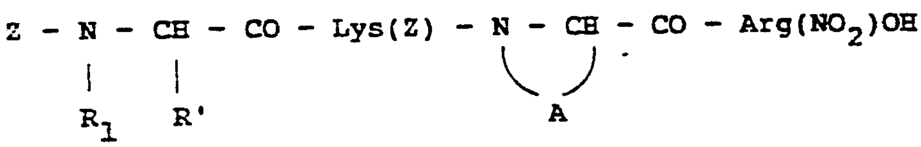

(X)

in which A, together with the carbon and nitrogen atoms to which it is attached, and $R_1$ have the same meanings as in formula I, and R' has the same meanings as in formula VIII, from which the protecting groups are then removed by hydrogenolysis to form a derivative of the formula I which can then, if desired :

be converted into a salt by a pharmaceutically acceptable acid or base,

be separated into its isomers, then, if necessary, converted into a salt by a pharmaceutically acceptable acid or base.

**Patentansprüche** (für die Vertragsstaaten : BE, CH, DE, FR, GB, IT, LI, LU, NL, SE)

1. Verbindungen der allgemeinen Formel I

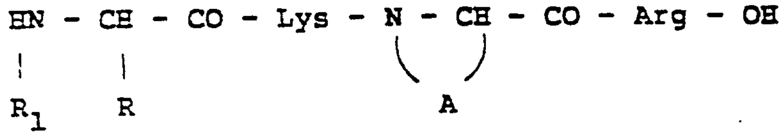

(I)

in der

R ein Wasserstoffatom, eine geradekettige oder verzweigte Alkylgruppe mit 1 bis 5 Kohlenstoffatomen, die gegebenenfalls durch eine Hydroxyl-, Amino-, Mercapto-, Methylthio-, Methylsulfoxid, Carboxy- oder Guanidino-Gruppe substituiert ist, oder eine gegebenenfalls durch eine Hydroxylgruppe substituierte Benzyl- oder Indol-3-yl-methylgruppe.

$R_1$ ein Wasserstoffatom oder eine Alkylgruppe mit 1 bis 4 Kohlenstoffatomen,

Lys und Arg jeweils den in Peptidbindung gebundenen Lysylrest bzw. Arginylrest und

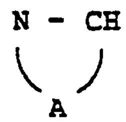

eine bicyclische Struktur der folgenden Formel

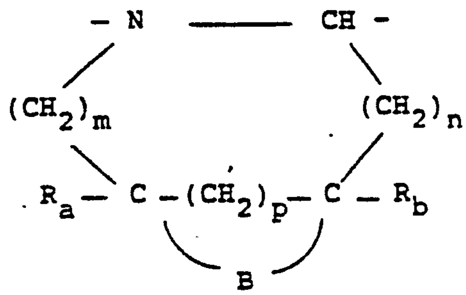

in der

m 1 oder Null,

n und p Null, 1 oder 2,

$R_a$ und $R_b$ Wasserstoffatome oder wenn p = Null ist, gemeinsam eine direkte Bindung, und

B eine Alkylenkette der Formel $(CH_2)_q$ worin q den Wert 2, 3 oder 4 besitzt, oder eine ungesättigte Gruppe der Formel $(—CH=CH—)_2$, wenn p = 0 und $R_a$ und $R_b$ gemeinsam eine Bindung bedeuten, mit der Maßgabe darstellen, daß die Summe von m, n, p und q eine ganze Zahl zwischen 3 und 6 darstellt, oder

die 1,2,3,4-Tetrahydro-betacarbolingruppe bedeuten,

deren Enantiomere, Epimere und Diastereoisomere,

sowie deren Additionssalze mit einer pharmazeutisch annehmbaren Säure oder Base.

2. Verbindungen nach Anspruch 1, dadurch gekennzeichnet, daß die cyclische Gruppe der Formel

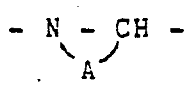

eine Indolin-, Isoindolin-, Tetrahydrochinolin-, Tetrahydroisochinolin-, Perhydroindol-, Perhydroisoindol-, Perhydroisochinolin-, Perhydrochinolin-, Perhydrocyclopenta [b]-pyrrol, 2-Aza-bicyclo [2.2.2] octan-, 2-Aza-bicyclo [2.2.1] heptan- oder 1,2,3,4-Tetrahydro-betacarbolin-Gruppe bedeutet, sowie die Enantiomeren, Epimeren und Diastereoisomeren dieser Verbindungen sowie deren Säureadditionssalze mit einer pharmazeutisch annehmbaren Säure oder Base.

3. Verbindungen der Ansprüche 1 und 2, worin R ein Wasserstoffatom oder eine gegebenenfalls durch eine Hydroxylgruppe substituierte geradkettige oder verzweigte Alkylgruppe mit 1 bis 5 Kohlenstoffatomen bedeutet, sowie die Enantiomeren, Epimeren und Diastereoisomeren dieser Verbindungen sowie deren Additionssalze mit einer pharmazeutisch annehmbaren Säure oder Base.

4. (S)Thr—(S)Lys—(S)PHI—(S)Arg—OH und dessen Additionssalze mit einer pharmazeutisch annehmbaren Säure oder Base.

5. (S)Thr—(S)Lys—(S)ABO—(S)Arg—OH und dessen Additionssalze mit einer pharmazeutisch annehmbaren Säure oder Base.

6. Gly—(S)Lys—(S)PHI—(S)Arg—OH und dessen Additionssalze mit einer pharmazeutisch annehmbaren Säure oder Base.

7. Gly—(S)Lys—(S)ABO—(S)Arg—OH und dessen Additionssalze mit einer pharmazeutisch annehmbaren Säure oder Base.

8. (S)NVa—(S)Lys—(S)PHI—(S)Arg—OH und dessen Additionssalze mit einer pharmazeutisch annehmbaren Säure oder Base.

9. (S)NVa—(S)Lys—(S)ABO—(S)Arg—OH und dessen Additionssalze mit einer pharmazeutisch annehmbaren Säure oder Base.

10. Sar—(S)Lys—(S)ABO—(S)Arg—OH und dessen Additionssalze mit einer pharmazeutisch annehmbaren Säure oder Base.

11. Verfahren zur Herstellung der Verbindungen der allgemeinen Formel I, dadurch gekennzeichnet, daß man ein Derivat der allgemeinen Formel II

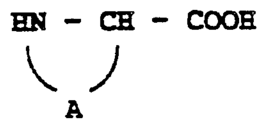

(II)

in der A zusammen mit dem Kohlenstoffatom und Stickstoffatom, an die es gebunden ist, die gleichen Bedeutungen wie bezüglich der allgemeinen Formel I angegeben besitzt, unter der Einwirkung von tert.-Butylcarbonat durch eine tert.-Butoxycarbonylgruppe (tBoc) unter Bildung eines Derivats der allgemeinen Formel III

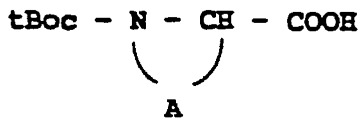

(III)

in der A zusammen mit dem Kohlenstoffatom und dem Stickstoffatom, an die es gebunden ist, die bezüglich der allgemeinen Formel I angegebenen Bedeutungen besitzt, schützt, welches man mit Methyl-N-ω-nitroarginat ($Arg(NO_2)OCH_3$) unter Bildung eines Derivats der allgemeinen Formel IV

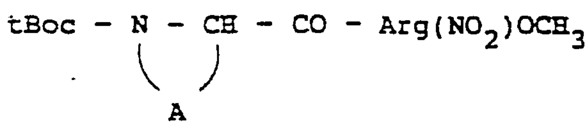

(IV)

worin A zusammen mit dem Kohlenstoffatom und dem Stickstoffatom, an die es gebunden ist, die gleichen Bedeutungen besitzt, wie bezüglich der allgemeinen Formel I angegeben, kondensiert, wonach man die Schutzgruppe mit Trifluoressigsäure unter Bildung eines Derivats der allgemeinen Formel V

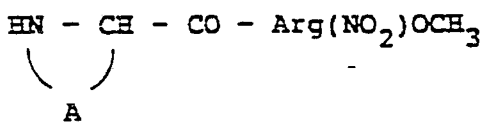

(V)

worin A, zusammen mit dem Kohlenstoffatom und dem Stickstoffatom, an die es gebunden ist, die gleichen Bedeutungen besitzt, wie bezüglich der allgemeinen Formel I angegeben, abspaltet, und anschließend mit N-tert.-Butoxy-carbonyl-N-ω-benzyloxycarbonyl-lysin oder (tBoc)Lys(Z) unter Bildung einer Verbindung der allgemeinen Formel VI

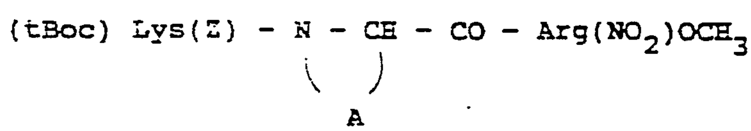

(VI)

worin A, zusammen mit dem Kohlenstoffatom und dem Stickstoffatom, an die es gebunden ist, die gleichen Bedeutungen besitzt, wie bezüglich der allgemeinen Formel I angegeben, kondensiert, die man unter der Einwirkung von Trifluoressigsäure zu einem Derivat der allgemeinen Formel VII

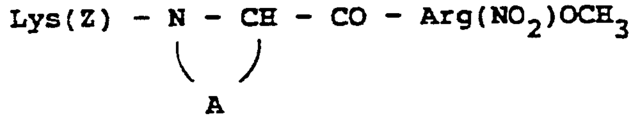

(VII)

worin A, zusammen mit dem Kohlenstoffatom und dem Stickstoffatom, an die es gebunden ist, die gleichen Bedeutungen besitzt, wie bezüglich der allgemeinen Formel I angegeben, umsetzt, welches man mit einem Derivat der allgemeinen Formel VIII

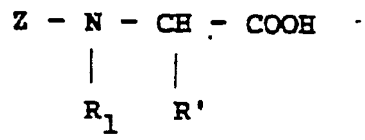 (VIII)

worin

Z eine Schutzgruppe für die Aminogruppen, insbesondere die Benzyloxycarbonylgruppe darstellt ;

$R_1$ die bezüglich der allgemeinen Formel I angegebenen Bedeutungen besitzt, und

R' ein Wasserstoffatom, eine Benzyl- oder Indol-3-yl-methyl-Gruppe, die gegebenenfalls durch eine Hydroxylgruppe substituiert ist, oder eine geradkettige oder verzweigte Alkylgruppe mit 1 bis 5 Kohlenstoffatomen, die gegebenenfalls durch eine Hydroxylgruppe, eine Mercaptogruppe, eine Methylthiogruppe, eine N-Nitrogruppe oder eine N,N-Bisbenzyloxycarbonyl-guanidino-Gruppe substituiert ist, oder eine Benzylcarboxylatgruppe bedeutet,

unter Bildung eines Derivats der allgemeinen Formel IX

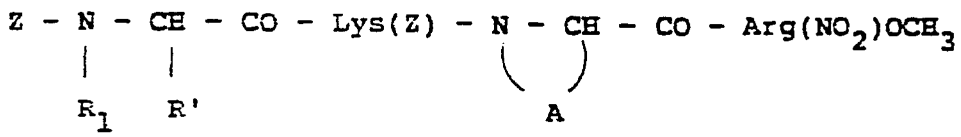 (IX)

in der A, zusammen mit dem Kohlenstoffatom und dem Stickstoffatom, an die es gebunden ist, und $R_1$ die bezüglich der allgemeinen Formel I angegebenen Bedeutungen besitzen und R' die bezüglich der allgemeinen Formel VIII angegebenen Bedeutungen besitzt, kondensiert, welches man zu einem Derivat der allgemeinen Formel X

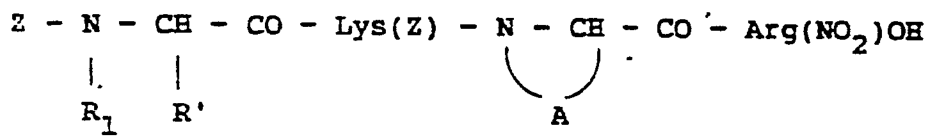 (X)

worin A zusammen mit dem Kohlenstoffatom und dem Stickstoffatom, an die es gebunden ist, und $R_1$ die bezüglich der allgemeinen Formel I angegebenen Bedeutungen besitzen und R' die bezüglich der allgemeinen Formel VIII angegebenen Bedeutungen besitzt, verseift, von welchem man anschließend die Schutzgruppen durch Hydrogenolyse abspaltet unter Bildung eines Derivats der allgemeinen Formel I, welches man gewünschtenfalls

entweder mit einer pharmazeutisch annehmbaren Säure oder Base in ein Salz überführen oder

in die Isomeren auftrennen und erforderlichenfalls mit einer pharmazeutisch annehmbaren Säure oder Base in ein Salz überführen kann.

12. Verbindungen der allgemeinen Formel IX nach Anspruch 11 zur Verwendung bei der Herstellung der Verbindungen nach Anspruch 1.

13. Pharmazeutische Zubereitung enthaltend als Wirkstoff mindestens eine Verbindung nach einem der Ansprüche 1 bis 10 in Kombination mit einem oder mehreren inerten, nicht-toxischen, pharmazeutisch annehmbaren Trägermaterialien oder Hilfsmitteln.

**Patentanspruch** (für den Vertragsstaat AT)

Verfahren zur Herstellung von Verbindungen der allgemeinen Formel I

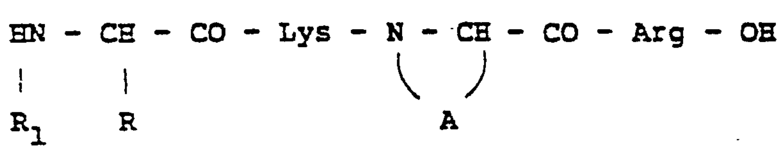 (I)

in der

R

ein Wasserstoffatom,

eine geradekettige oder verzweigte Alkylgruppe mit 1 bis 5 Kohlenstoffatomen, die gegebenenfalls durch eine Hydroxyl-, Amino-, Mercapto-, Methylthio-, Methylsulfoxid, Carboxy- oder Guanidino-Gruppe substituiert ist, oder

eine gegebenenfalls durch eine Hydroxylgruppe substituierte Benzyl- oder Indol-3-yl-methylgruppe. $R_1$ ein Wasserstoffatom oder eine Alkylgruppe mit 1 bis 4 Kohlenstoffatomen, Lys und Arg jeweils den in Peptidbindung gebundenen Lysylrest bzw. Arginylrest und

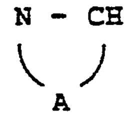

eine bicyclische Struktur der folgenden Formel

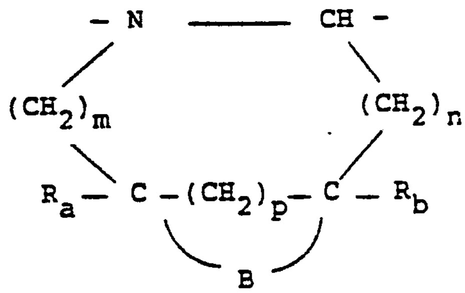

in der

m 1 oder Null,

n und p Null, 1 oder 2,

$R_a$ und $R_b$ Wasserstoffatome oder wenn p = Null ist, gemeinsam eine direkte Bindung, und

B eine Alkylenkette der Formel $(CH_2)_q$ worin q den Wert 2, 3 oder 4 besitzt, oder eine ungesättigte Gruppe der Formel (—CH=CH—)$_2$, wenn p = 0 und $R_a$ und $R_b$ gemeinsam eine Bindung bedeuten, mit der Maßgabe darstellen, daß die Summe von m, n, p und q eine ganze Zahl zwischen 3 und 6 darstellt, oder

die 1,2,3,4-Tetrahydro-betacarbolingruppe bedeuten, und deren Enantiomere, Epimere und Diastereoisomere, sowie deren Additionssalze mit einer pharmazeutisch annehmbaren Säure oder Base, dadurch gekennzeichnet, daß mann ein Derivat der allgemeinen Formel II

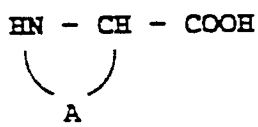

(II)

in der A zusammen mit dem Kohlenstoffatom und Stickstoffatom, an die es gebunden ist, die gleichen Bedeutungen wie bezüglich der allgemeinen Formel I angegeben besitzt, unter der Einwirkung von tert.-Butylcarbonat durch eine tert.-Butoxycarbonylgruppe (tBoc) unter Bildung eines Derivats der allgemeinen Formel III

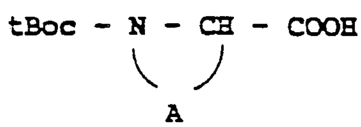

(III)

in der A zusammen mit dem Kohlenstoffatom und dem Stickstoffatom, an die es gebunden ist, die bezüglich der allgemeinen Formel I angegebenen Bedeutungen besitzt, schützt, welches man mit Methyl-N-ω-nitroarginat ($Arg(NO_2)OCH_3$) unter Bildung eines Derivats der allgemeinen Formel IV

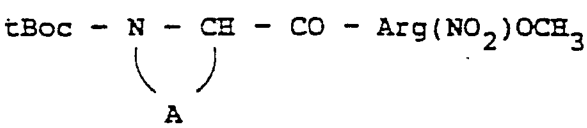

(IV)

worin A zusammen mit dem Kohlenstoffatom und dem Stickstoffatom, an die es gebunden ist, die gleichen Bedeutungen besitzt, wie bezüglich der allgemeinen Formel I angegeben, kondensiert, wonach man die Schutzgruppe mit Trifluoressigsäure unter Bildung eines Derivats der allgemeinen Formel V

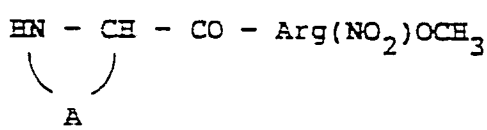

(V)

worin A, zusammen mit dem Kohlenstoffatom und dem Stickstoffatom, an die es gebunden ist, die gleichen Bedeutungen besitzt, wie bezüglich der allgemeinen Formel I angegeben, abspaltet, und anschließend mit N-tert.-Butoxy-carbonyl-N-ω-benzyloxycarbonyl-lysin oder (tBoc)Lys(Z) unter Bildung einer Verbindung der allgemeinen Formel VI

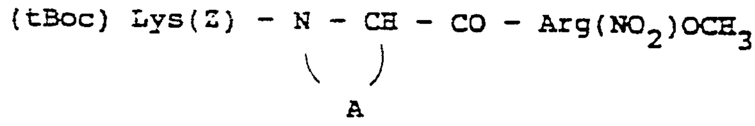
(VI)

worin A, zusammen mit dem Kohlenstoffatom und dem Stickstoffatom, an die es gebunden ist, die gleichen Bedeutungen besitzt, wie bezüglich der allgemeinen Formel I angegeben, kondensiert, die man unter der Einwirkung von Trifluoressigsäure zu einem Derivat der allgemeinen Formel VII

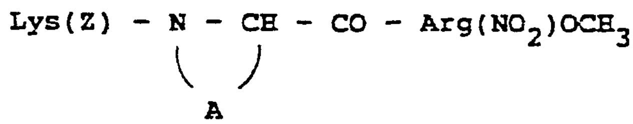
(VII)

worin A, zusammen mit dem Kohlenstoffatom und dem Stickstoffatom, an die es gebunden ist, die gleichen Bedeutungen besitzt, wie bezüglich der allgemeinen Formel I angegeben, umsetzt, welches man mit einem Derivat der allgemeinen Formel VIII

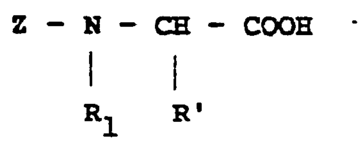
(VIII)

worin

Z eine Schutzgruppe für die Aminogruppen, insbesondere die Benzyloxycarbonylgruppe darstellt ;

$R_1$ die bezüglich der allgemeinen Formel I angegebenen Bedeutungen besitzt, und

R′ ein Wasserstoffatom, eine Benzyl- oder Indol-3-yl-methyl-Gruppe, die gegebenenfalls durch eine Hydroxylgruppe substituiert ist, oder eine geradkettige oder verzweigte Alkylgruppe mit 1 bis 5 Kohlenstoffatomen, die gegebenenfalls durch eine Hydroxylgruppe, eine Mercaptogruppe, eine Methylthiogruppe, eine N-Nitrogruppe oder eine N,N-Bisbenzyloxycarbonyl-guanidino-Gruppe substituiert ist, oder eine Benzylcarboxylatgruppe bedeutet,

unter Bildung eines Derivats der allgemeinen Formel IX

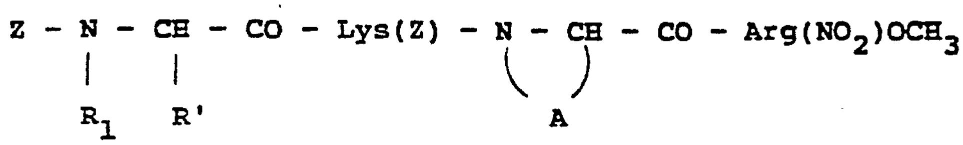
(IX)

in der A, zusammen mit dem Kohlenstoffatom und dem Stickstoffatom, an die es gebunden ist, und $R_1$ die bezüglich der allgemeinen Formel I angegebenen Bedeutungen besitzen und R′ die bezüglich der allgemeinen Formel VIII angegebenen Bedeutungen besitzt, kondensiert, welches man zu einem Derivat der allgemeinen Formel X

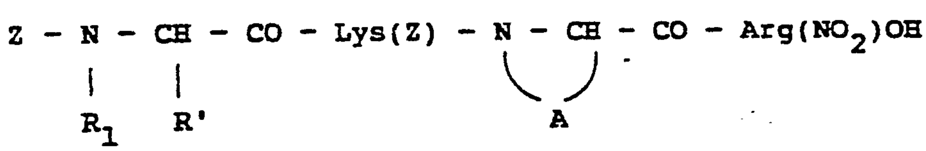
(X)

worin A zusammen mit dem Kohlenstoffatom und dem Stickstoffatom, an die es gebunden ist, und $R_1$ die bezüglich der allgemeinen Formel I angegebenen Bedeutungen besitzen und R′ die bezüglich der allgemeinen Formel VIII angegebenen Bedeutungen besitzt, verseift, von welchem man anschließend die Schutzgruppen durch Hydrogenolyse abspaltet unter Bildung eines Derivats der allgemeinen Formel I, welches man gewünschtenfalls

entweder mit einer pharmazeutisch annehmbaren Säure oder Base in ein Salz überführen oder

in die Isomeren auftrennen und erforderlichenfalls mit einer pharmazeutisch annehmbaren Säure oder Base in ein Salz überführen kann.